# FASCICULE DE BREVET EUROPEEN

(11) **EP 1 750 707 B1**
(45) Date de publication et mention de la délivrance du brevet: **14.03.2018**
(21) Numéro de dépôt: 04816441.2
(22) Date de dépôt: 20.12.2004
(51) Int. Cl.: A61K 31/4745, A61K 31/685, A61P 37/04, A61K 39/00

(54) **Composition immunostimulante comprenant au moins un agoniste du récepteur Toll-like 7 ou du récepteur Toll-like 8 et un agoniste du récepteur Toll-like 4**
Immunstimulierende Zusammensetzung mit mindestens einem Agonisten des Toll-like 7 Rezeptors oder des Toll-like 8 Rezeptors und einem Toll-like 4 Rezeptor Agonisten
Immunostimulant composition comprising at least one agonist of the Toll-like 7 receptor or of the Toll-like 8 receptor and one Toll-like 4 receptor agonist

(30) Priorité: 19.12.2003 FR 0314995
(43) Date de publication de la demande: 14.02.2007
(73) Titulaire: Sanofi Pasteur, 69007 Lyon (FR)
(72) Inventeur: BURDIN, Nicolas, F-69130 Ecully (FR); HAENSLER, Jean, Valency 69290 Poillonnay (FR)
(74) Mandataire: Kerneis, Daniéle
(86) Numéro de dépôt international: PCT/FR2004/003308
(87) Numéro de publication internationale: WO 2005/060966

(56) Documents cités:
- WO-A-03/043572
- WO-A-03/086280
- WO-A-2004/060319
- WO-A2-2005/025614
- WO-A2-2006/068663
- US-A1- 2003 139 364
- VASILAKOS J P ET AL: "ADJUVANT ACTIVITIES OF IMMUNE RESPONSE MODIFIER R-848: COMPARISON WITH CPG ODN" CELLULAR IMMUNOLOGY, ACADEMIC PRESS, SAN DIEGO, CA, US, vol. 204, no. 1, 25 août 2000 (2000-08-25), pages 64-74, XP001037913 ISSN: 0008-8749
- PRZETAK, MELINDA ET AL: "Novel synthetic LPS receptor agonists boost systemic and mucosal antibody responses in mice" VACCINE , 21(9-10), 961-970 CODEN: VACCDE; ISSN: 0264-410X, 2003, XP002288914 cité dans la demande
- AKIRA S ED - EISENLOHR LAURENCE C SCHUURMAN JANINE PARREN PAUL WHI: "Mammalian Toll-like receptors", CURRENT OPINION IN IMMUNOLOGY, ELSEVIER, OXFORD, GB, vol. 15, no. 1, 1 February 2003 (2003-02-01), pages 5-11, XP004399365, ISSN: 0952-7915, DOI: 10.1016/S0952-7915(02)00013-4
- GORDEN KEITH B ET AL: "Synthetic TLR agonists reveal functional differences between human TLR7 and TLR8", THE JOURNAL OF IMMUNOLOGY, THE AMERICAN ASSOCIATION OF IMMUNOLOGISTS, US, vol. 174, no. 3, 1 February 2005 (2005-02-01), pages 1259-1268, XP009163641, ISSN: 0022-1767, DOI: 10.4049/JIMMUNOL.174.3.1259
- , Retrieved from the Internet: URL:https://en.wikipedia.org/wiki/Toll-lik e_receptor [retrieved on 2017-04-24]

## Description

L'invention est relative au domaine des compositions immunostimulantes comprenant au moins un agoniste du récepteur Toll-like 7 ou du récepteur Toll-like 8 qui sont présents sur les cellules présentatrices d'antigènes. Plus particulièrement, l'invention concerne des compositions qui comprennent en outre un agoniste du récepteur Toll-like 4, et notamment de telles compositions qui comprennent en outre un antigène vaccinal. Il est connu dans l'art antérieur de vouloir augmenter ou orienter la réponse immunitaire induite par les antigènes présents dans un vaccin, au moyen d'adjuvants qui sont choisis dans la catégorie des immunostimulants. Ceci peut être souhaitable car l'antigène, administré seul, n'est pas suffisamment immunogène, à cause notamment de son très haut degré de pureté, ou parce qu'on souhaite réduire la quantité d'antigènes présents dans le vaccin ou le nombre de rappels à effectuer, ou encore parce qu'on souhaite allonger la durée de protection conférée par le vaccin. Parfois, il s'agit de modifier qualitativement plutôt que quantitativement la réponse induite.

De nombreuses molécules ont déjà été décrites en relation avec leurs propriétés adjuvantes ; cependant, les principaux adjuvants actuellement commercialisés dans les vaccins sont des adjuvants à base d'aluminium ou des émulsions.

Ainsi, parmi l'art antérieur connu, on peut notamment citer le brevet EP636031, qui divulgue l'utilisation d'une 1H-imidazo[4,5c]quinoléine-4-amine comme adjuvant vaccinal, vis-à-vis d'une glycoprotéine du virus Herpes Simplex 2 chez le cobaye. Dans ce document, le vaccin administré ne permet pas d'empêcher complètement le développement de la maladie, lors d'un challenge des animaux par du virus HSV2, mais il permet de réduire les lésions, l'excrétion vaginale du virus ainsi que le phénomène de récurrence de la maladie.

Selon la publication intitulée « Adjuvant activities of Immune Response Modifier R848 : Comparison with CpG ODN », de Vasilakos et al, dans Cellular Immunology 204, 64-74 (2000), le dérivé d'imidazoquinoléine R-848 est décrit pour être un adjuvant de type TH1, dans un test utilisant comme antigène l'ovalbumine administrée à des souris. Cette publication décrit également un autre type d'adjuvant vaccinal constitué par des oligonucléotides comprenant un dinucléotide CG, dans lequel la cytosine n'est pas méthylée.

La demande WO03/086280 décrit des molécules d'ARN immunostimulantes qui agiraient par interaction avec des TLR8, des TLR7, ou d'autres TLR et auxquelles il est possible d'ajouter un autre ligand de TLR.

Dans un autre document de l'art antérieur constitué par la publication intitulée *"*Human TLR7 or TLR8 independently confer responsiveness to the antiviral compound R-848" de Jurk et al, dans Nature Immunology, June 2002, volume 3 n°6, p499, il est indiqué que les récepteurs Toll-like jouent un rôle important dans les réponses immunes aux pathogènes, le récepteur Toll-like 9 étant activé par l'ADN bactérien ayant des motifs CpG non méthylés, alors que le R-848 active les cellules via le récepteur Toll-like 7 et le récepteur Toll-like 8.

Dans la publication intitulée « Novel synthetic LPS receptor agonists boost systemic and mucosal antibody responses in mice », de Przetak et al, dans Vaccine 21 (2003) pages 961-970, il est décrit des composés chimiques ayant des chaînes d'acides gras, composés dépourvus de noyaux sucre mais qui ont une activité adjuvante vis-à-vis d'antigènes constitués par la toxine tétanique ou l'ovalbumine. Ces composés sont connus pour activer un mécanisme d'action lié au récepteur Toll-like 4.

Tous ces composés sont connus pour avoir, individuellement, des propriétés immunostimulantes à des degrés divers selon les conditions d'administration; cependant, il reste souhaitable de pouvoir disposer d'une composition permettant de potentialiser ces propriétés immunostimulantes, en particulier dans le cas de l'administration d'un antigène vaccinal.

Pour atteindre cet objectif la présente invention a pour objet une composition immunostimulante comprenant au moins un agoniste du récepteur Toll-like 7 ou du récepteur Toll-like 8, ainsi qu'un agoniste du récepteur Toll-like 4, caractérisée en ce que :
- l'agoniste du récepteur Toll-like 7 ou du récepteur Toll-like 8 est un dérivé d'imidazoquinoléine amine,
- l'agoniste du récepteur Toll-like 4 est le ER804057, et
en ce qu'elle comprend en outre un antigène vaccinal autre qu'un ADN codant.

Ainsi, on obtient une potentialisation de la réponse immunostimulante.

La réponse immune induite vis-à-vis de l'antigène est potentialisée.

Selon l'invention, l'agoniste du récepteur Toll-like 7 ou du récepteur Toll-like 8 est un dérivé d'imidazoquinoléine amine. Un tel agoniste peut être obtenu par synthèse chimique pure et présente donc toutes les garanties de reproductibilité et de sécurité nécessaires à un usage pharmaceutique.

Selon un mode particulier de réalisation, le dérivé d'imidazoquinoléine amine est le 4-amino-2-ethoxymethyl-α, α, dimethyl-1-H-imidazo[4,5c]quinoleine-1-ethanol.

Selon l'invention, l'agoniste du récepteur Toll-like 4 est un composé décrit dans la demande WO0044758 : le ER804057 ; un tel composé, obtenu par pure synthèse chimique présente également toutes les garanties de reproductibilité et de sécurité nécessaire à un usage pharmaceutique.

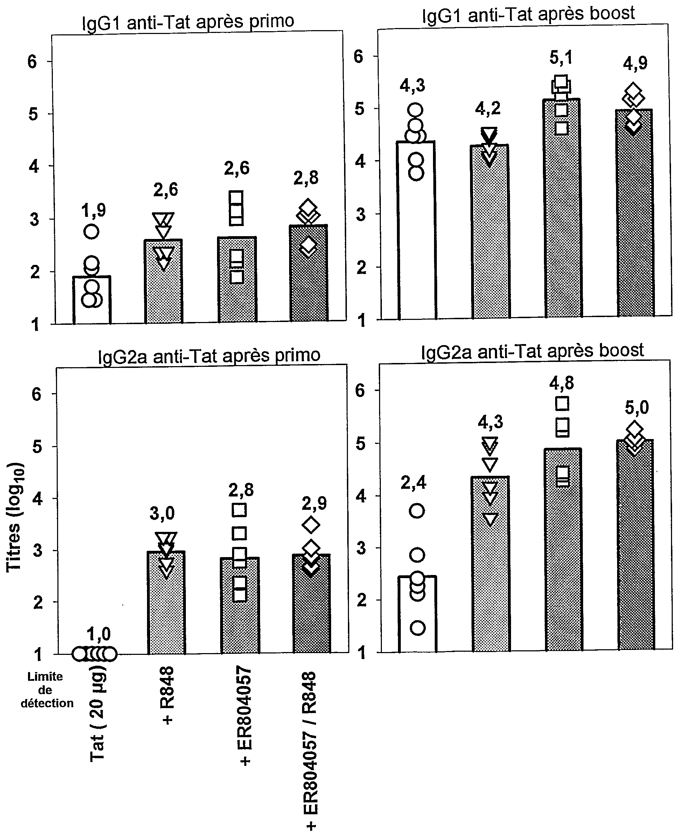

De nombreux autres avantages de la présente invention apparaîtront à la lumière de la description détaillée qui va suivre, en référence aux figures 1 à 4 qui illustrent les résultats obtenus à l'exemple 5.

La présente invention est relative à une composition immunostimulante ; par composition immunostimulante, on entend une composition susceptible d'induire la maturation ou l'activation de cellules du système immunitaire, telles que les cellules dendritiques, ce qui conduit alors à l'expression sur les cellules de certains marqueurs ( CD25, CD80, CD83 ou autres) que l'on peut détecter, et à la sécrétion de cytokines (IL6, IL12p70, TNF-α,...) que l'on peut doser.

Selon un mode particulier de réalisation, la composition immunostimulante de l'invention comprend au moins un antigène vaccinal. Par antigène vaccinal, on entend un antigène susceptible d'induire une réponse du système immunitaire lorsqu'il est administré à l'homme ou à un animal. Cette réponse du système immunitaire peut se traduire par une production d'anticorps ou par une activation de certaines cellules, notamment les cellules présentatrices d'antigènes (ex : les cellules dendritiques), les lymphocytes T, les lymphocytes B.

La composition vaccinale peut être une composition à visée prophylactique ou à visée thérapeutique, ou encore les deux.

Elle peut être administrée par toutes les voies habituellement utilisées ou préconisées pour les vaccins : voie parentérale, voie muqueuse, et se présenter sous diverses formes : liquide injectable ou pulvérisable, formulation lyophilisée ou séchée par atomisation ou à l'air,...etc. Elle peut être administrée au moyen d'une seringue ou au moyen d'un injecteur sans aiguille pour injection intramusculaire, sous-cutanée ou intradermique. Elle peut aussi être administrée grâce à un nébuliseur capable de délivrer une poudre sèche ou un spray liquide au niveau des muqueuses, qu'elles soient nasales, pulmonaires, vaginales ou rectales.

Les antigènes vaccinaux utilisés dans les compositions vaccinales selon la présente invention sont des antigènes « directs », c'est-à-dire qu'il ne s'agit pas d'ADN codant pour ces antigènes, mais les antigènes eux-mêmes ; il peut s'agir d'un germe entier ou seulement d'une partie de ce germe ; ainsi parmi les antigènes utilisés habituellement dans les vaccins, on peut notamment citer :
- les polysaccharides, qu'ils soient seuls ou conjugués à des éléments porteurs, tels que les protéines porteuses,
- les germes entiers vivants atténués,
- les germes inactivés,
- les peptides et protéines recombinants,
- les glycoprotéines, les glycolipides, les lipopeptides,
- les peptides synthétiques,
- les germes éclatés dans le cas de vaccins appelés vaccins « splittés ».

Ces antigènes sont des antigènes utilisés ou susceptibles d'être utilisés pour le traitement ou la prévention de diverses maladies telles que par exemple : diphtérie, tétanos, polio, rage, coqueluche, hépatites A, B, C, fièvre jaune, fièvre typhoïde, varicelle, rougeole, oreillons, rubéole, encéphalite japonaise, méningites, infections à pneumocoques, infections à rotavirus, SIDA, cancers, tuberculose, maladie de Lyme, infections à RSV, herpès, affections bactériennes provoquées par Chlamydia, Neisseria gonorrheae, Streptococcus pneumoniae, Moraxella catarrhalis, ou Haemophilus influenza type B, la malaria, les leshmanioses, les listerioses, ...etc.

La composition vaccinale selon l'invention peut être une composition destinée à l'immunisation contre un seul pathogène ou cancer, c'est-à-dire qu'elle comprend un ou plusieurs antigènes d'un seul pathogène ou cancer, ou bien être une composition destinée à l'immunisation contre plusieurs pathogènes ou cancers (on parle alors de combinaison vaccinale).

Au sens de la présente invention, on entend par agoniste des récepteurs Toll-like 7 et Toll-like 8, un composé capable de se lier à l'un ou l'autre de ces récepteurs ou aux deux et de déclencher la cascade de signalisation associée à ces récepteurs, notamment un composé capable d'activer la translocation de NF-κB dans des cellules transfectées avec du cDNA codant pour l'un ou l'autre de ces récepteurs, ou pour les deux.

Comme agonistes convenant aux fins de l'invention, on peut citer les imidazoquinoléines amines substituées, et notamment celles décrites dans le brevet US 5389640. On a obtenu de particulièrement bons résultats avec le 4-amino-2-ethoxymethyl-α, α, dimethyl-1-H-imidazo[4,5c]quinoleine-1-ethanol, encore appelé R-848 dont une méthode de préparation est indiquée aux exemples 99 et 101 de l'USP5389640.

Au sens de la présente invention, on entend par agoniste du récepteur Toll-like 4, un composé capable de se lier à ce récepteur et de déclencher la cascade de signalisation associée, notamment un composé capable d'activer la translocation de NF-κB dans des cellules transfectées avec du cDNA codant pour ce récepteur.

Parmi les agonistes du récepteur Toll-like 4, on connait les LPS des bactéries Gram négatives, des dérivés monophosphorylés des lipides A de ces LPS, et notamment, le 3D-MPL ou lipid A monophosphorylé deacylé en position 3 qui est décrit par RIBI dans le brevet UK N°2211502 ainsi que dans l'USP 4436727 et sa Reissue 4912094. Des analogues synthétiques de ces produits, tels que ceux décrits par CORIXA dans la demande WO98/50399, et notamment le RC-529, ou encore ceux décrits dans la demande WO02/12258 ont également ces propriétés. De même, les composés objets des demandes WO95/14026, WO00/00462, WO01/46126 et WO01/46127 au nom de OM Pharma, sont des agonistes du récepteur Toll-like 4.

Le brevet USP6290973 au nom de EISAI CO décrit des produits purement synthétiques, dépourvus de noyau saccharidique, tels que le produit dénommé ER112066, ou encore le produit dénommé ER804057 qui est le produit selon l'invention. Ce produit est un sel disodique de (1R,6R,22R,27R)-1,27-diheptyl-1,27-bis dodecanoyl -9,19-dihydroxy-9,19-dioxido-14-oxo-6,22-bis[(1,3-dioxotetradecyl)amino]-4,8,10,18,20,24-hexaoxa-13,15-diaza-9,19-diphosphoheptacosane qui peut être obtenu en suivant la méthode de préparation indiquée dans la demande de brevet WO0044758, pour le composé N° 50, i.e. la méthode décrite à la page 32, à condition de remplacer auparavant dans l'étape menant du produit 39 au produit 41 décrite à la page 28 de la demande de brevet, le chlorure de myristoyl par de l'acide β-cetomyristique en présence de EDC (c'est-à-dire de l'hydrochlorure de 1-(3-dimethylaminopropyl)-3-ethylcarbodiimide).

Chacun de ces agonistes, que ce soit l'agoniste du récepteur Toll-like 4 ou l'agoniste des récepteurs Toll-like 7 et 8 est connu pour avoir des propriétés immunostimulantes. L'intérêt de la présente invention est que la réponse observée dans le cadre de la présente invention est une réponse potentialisée. Si l'obtention d'une telle potentialisation pourrait pour une personne non familière avec le domaine de l'immunologie apparaître au 1^{er} abord comme évidente, il faut au contraire la considérer comme surprenante dans le domaine particulier de l'invention ; en effet, les expériences ont montré que, lorsque 2 ou plusieurs immunostimulants sont présents dans une même composition, il est fréquent que l'effet produit par l'un d'entre eux soit inhibiteur vis-à-vis de l'autre ou des autres immunostimulants présents, ou du moins lorsqu'un produit exerce un effet immunostimulant, il est difficile d'augmenter encore la réponse déjà obtenue.

Dans les tests d'immunostimulation in vitro, on observe une synergie des effets des immunostimulants mis au contact des cellules du système immunitaire qui induisent un niveau d'activation des cellules tout à fait exceptionnel, qui ne pouvait être anticipé à partir du niveau d'activation induit par chacun des immunostimulants utilisé séparément. De même, la synergie observée dans la réponse obtenue lorsque la composition immunostimulante comprend des antigènes vaccinaux, notamment en ce qui concerne le nombre de sujets répondeurs avec un très bon niveau de réponse, est tout à fait exceptionnelle, et ne pouvait nullement être déduite des réponses obtenues avec chacun des adjuvants pris isolément.

Les résultats obtenus en utilisant des agonistes de ces récepteurs Toll-like 7, 8 et 4 sont d'autant plus surprenants que des essais réalisés en combinant plusieurs agonistes d'autres récepteurs n'ont abouti à aucune potentialisation des effets observés par chacun des agonistes utilisé isolément.

Les agonistes des récepteurs Toll-like 4, 7 et 8 de la présente invention ont la propriété d'adjuver les antigènes vaccinaux avec lesquels ils sont administrés, ce qui en général signifie qu'ils sont capables d'accroître ou de modifier la réponse du système immunitaire de l'organisme auquel la composition vaccinale est administrée, par rapport à la réponse qui serait obtenue en leur absence. En particulier, il peut s'agir d'une augmentation de la réponse humorale, ou de la réponse cellulaire, ou des deux. L'action peut également être, non pas une augmentation de la réponse, mais une orientation différente de la réponse induite : par exemple, orientation vers une réponse cellulaire plutôt qu'une réponse humorale, production de certaines cytokines plutôt que d'autres, production de certains types ou sous-types d'anticorps plutôt que d'autres, stimulation de certaines cellules plutôt que d'autres, etc... L'action d'un adjuvant peut également consister à augmenter la durée de la réponse immune dans le temps. Il peut aussi s'agir de permettre la diminution du nombre d'administrations nécessaires pour obtenir une protection de l'individu immunisé, ou encore la diminution de la quantité d'antigènes contenue dans la dose administrée.

Dans le cas de la présente invention, la synergie observée se traduit essentiellement par une diminution de la dispersion des résultats obtenus, notamment en ce qui concerne la réponse Th1.

L'action adjuvante des agonistes selon l'invention peut être obtenue, soit lorsqu'ils sont associés à l'antigène ou aux antigènes de la composition vaccinale lors de leur administration, i.e. lorsqu'ils sont présents directement dans la composition vaccinale, ou encore lorsqu'ils sont administrés séparément de l'antigène ou des antigènes dont on veut modifier le pouvoir immunogène. On préfère cependant les utiliser dans la même composition vaccinale que l'antigène ou les antigènes à administrer.

Les exemples qui suivent illustrent des modes de réalisation particuliers de la présente invention.

### 1. Préparation d'une suspension stock d'agonistes des récepteurs Toll-like 7 et 8.

On dispose de dipalmitoylphosphatidylcholine (DPPC) obtenue chez Avanti Polar Lipids (Alabaster, AL), et de 4-amino-2-ethoxymethyl-α, α, dimethyl-1-H-imidazo[4,5c]quinoleine-1-ethanol (R-848) fourni par la Société InVivogen. Ces composés se présentent sous forme de poudre.

342 µg de DPPC (0.46 µmoles) additionnés de 150 µg de R-848 (0.51 µmoles) sont dissous dans 984 µl d'un mélange chloroforme/méthanol 4 :1 (vol/vol). La solution est séchée dans un ballon en verre à l'aide d'un évaporateur rotatif de manière à laisser un film lipidique homogène sur les parois du ballon. Ce film est encore séché sous vide poussé pour éliminer toute trace de solvant résiduel puis repris dans 3 ml d'eau à 60°C. La suspension liposomale résultante est homogénéisée par agitation au vortex, sonication dans un bain à ultrasons puis extrudée séquentiellement, à l'aide d'un extrudeur Lipex thermostaté à 50°C, en un passage à travers une membrane de polycarbonate de porosité 0.8 µm, puis un passage à travers une membrane de porosité 0.4 µm et enfin un passage à travers une membrane de porosité 0.2 µm.

On obtient ainsi des liposomes de DPPC/R-848 (0.9 :1 mol/mol) en eau à 114 µg/ml de DPPC et 50 µg/ml de R-848.

### 2. Préparation d'une suspension stock d'agonistes de récepteur Toll-like 4.

On dispose de dipalmitoylphosphatidylcholine (DPPC) obtenue chez Avanti Polar Lipids (Alabaster, AL) et de ER804057 fourni par la Société Eisai.

Ces composés se présentent sous forme de poudre.

273 µg de DPPC (0.37 µmoles) additionnés de 150 µg de ER804057 (0.092 µmoles) sont dissous dans 760 µl d'un mélange chloroforme/méthanol 4 :1 (vol/vol). La solution est séchée dans un ballon en verre à l'aide d'un évaporateur rotatif de manière à laisser un film lipidique homogène sur les parois du ballon. Ce film est encore séché sous vide poussé pour éliminer toute trace de solvant résiduel puis repris dans 3 ml d'eau à 60°C. La suspension liposomale résultante est homogénéisée par agitation au vortex, sonication dans un bain à ultrasons puis extrudée séquentiellement, à l'aide d'un extrudeur Lipex thermostaté à 50°C, en un passage à travers une membrane de polycarbonate de porosité 0.8 µm, puis un passage à travers une membrane de porosité 0.4 µm et enfin un passage à travers une membrane de porosité 0.2 µm.

On obtient ainsi des liposomes de DPPC/ER804057 (4 :1 mol/mol) en eau à 91 µg/ml de DPPC et 50 µg/ml de ER804057.

### 3. Préparation d'une suspension stock d'agonistes de récepteur Toll-like 4 et d'agonistes de récepteurs Toll-like 7 et 8.

On dispose de dipalmitoylphosphatidylcholine (DPPC) obtenue chez Avanti Polar Lipids (Alabaster, AL), de 4-amino-2-ethoxymethyl-α, α, dimethyl-1-H-imidazo[4,5c]quinoleine-1-ethanol (R-848) fourni par la Société InVivogen, et de ER804057 fourni par la Société Eisai.

Ces composés se présentent sous forme de poudre.

273 µg de DPPC (0.37 µmoles) additionnés de 150 µg de TLA4 (0.092 µmoles) et de 150 µg de R848 (0.51 µmoles) sont dissous dans 1.06 ml d'un mélange chloroforme/méthanol 4 :1 (vol/vol). La solution est séchée dans un ballon en verre à l'aide d'un évaporateur rotatif de manière à laisser un film lipidique homogène sur les parois du ballon. Ce film est encore séché sous vide poussé pour éliminer toute trace de solvant résiduel puis repris dans 3 ml d'eau à 60°C. La suspension liposomale résultante est homogénéisée par agitation au vortex, sonication dans un bain à ultrasons puis extrudée séquentiellement, à l'aide d'un extrudeur Lipex thermostaté à 50°C, en un passage à travers une membrane de polycarbonate de porosité 0.8 µm, puis un passage à travers une membrane de porosité 0.4 µm et enfin un passage à travers une membrane de porosité 0.2 µm.

On obtient ainsi des liposomes de DPPC/ER804057/R-848 (4 :1 : 5.5 mol/mol/mol) en eau à 91 µg/ml de DPPC, 50 µg/ml de ER804057 et 50 µg/ml de R-848.

### 4. Préparation des compositions vaccinales

On prépare des compositions vaccinales comprenant à titre d'antigène vaccinal, une protéine recombinante susceptible d'être utilisée dans un vaccin contre le SIDA ; il s'agit de la protéine TAT III B détoxifiée qui est obtenue par expression chez E. coli et purification par différentes étapes de chromatographie, puis inactivation chimique, ainsi que cela est décrit dans la demande WO99/33346, où elle est identifiée sous le terme de Tat carboxyméthylée.

Les compositions sont préparées de la manière décrite ci-après.

Les suspensions liposomales préparées selon les exemples 1 à 3 sont mélangées volume à volume (0.9 ml + 0.9 ml) à une solution de Tat concentrée à 200 µg/ml en tampon TRIS 100 mM, NaCl 200 mM, pH 7.4 pour obtenir les préparations (1.8 ml final) dont la composition est indiquée ci-après et dans lesquelles les quantités d'antigènes et d'adjuvant sont indiquées par dose de 200 µl.
1) Tat (20 µg)
2) Tat (20 µg) + ER804057/DPPC (5 µg / 9.1 µg soit 3.1 nmoles / 12.4 nmoles)
3) Tat (20 µg) + ER804057/DPPC/R-848 (5 µg / 9.1 µg / 5 µg soit 3.1 nmoles / 12.4 nmoles / 16.7 nmoles)
4) Tat (20 µg) + R-848/DPPC (5 µg /11.4 µg soit 16.7 nmoles /15.5 nmoles).

### 5. Test d'immunisation sur souris.

On dispose de 4 groupes de 6 souris BALB/c femelles âgées de 8 semaines, à qui l'on injecte une des compositions préparées à l'exemple 4, par voie sous-cutanée, à raison d'une dose de 200µl par souris ; les injections sont réalisées à J0 et à J21.

On prélève des échantillons sanguins au sinus retro-orbital à J14 pour l'appréciation de la réponse primaire et à J32 pour la réponse secondaire. La détermination du taux d'IgG1 et d'IgG2a spécifiques est effectuée grâce à des tests ELISA standardisés. Les souris sont sacrifiées à J37 ; on prélève leur rate et on isole les splénocytes.

Les résultats obtenus en ce qui concerne les réponses humorales sont récapitulés dans le tableau ci-dessous et aux Figures 1 à 4, où les taux d'IgG sont exprimés en unités arbitraires ELISA (log10).

Pour chaque groupe de souris, la valeur indiquée dans le tableau est le titre géométrique moyen des valeurs obtenues pour chacune des souris.

| Composition vaccinale | IgG1 à J14 | IgG2a à J14 | IgG1 à J32 | IgG2a à J32 | Rapport IgG1/IgG2a à J32 |
|---|---|---|---|---|---|
| Tat | 1,897 | 1,000 | 4,343 | 2,436 | 176,2 |
| Tat+ ER804057 | 2,598 | 2,820 | 5,101 | 4,838 | 3,5 |
| Tat + R848 | 2,568 | 2,959 | 4,248 | 4,328 | 1,3 |
| Tat + ER804057 +R848 | 2,805 | 2,864 | 4,877 | 4,989 | 0,9 |

Le rapport IgG1/IgG2a permet d'apprécier l'orientation de la réponse immunitaire induite. En effet, une réponse de type Th1 se traduit chez la souris par une plus grande proportion d'IgG2a, alors qu'une réponse de type Th2 se traduit par une plus grande proportion d'IgG1.

On voit donc que, grâce à la composition selon l'invention, la réponse est orientée vers le type Th1, bien plus fortement que si chacun des immunostimulants était utilisé individuellement.

Les graphes représentés sur les figures 1 à 4 permettent de visualiser les réponses obtenues pour chacune des souris, et donc d'apprécier la plus ou moins grande dispersion des résultats. Les performances de la composition selon l'invention sont particulièrement notables au niveau de la réponse en IgG2a obtenue après l'injection de rappel ; en effet, si les niveaux de réponse obtenus avec les compositions ayant un seul immunostimulant, que ce soit le R-848 ou l'ER804057, sont en moyenne satisfaisantes, on remarque que les résultats sont dans ces cas relativement dispersés ; alors qu'avec la composition selon l'invention, toutes les souris ont produit un taux d'IgG2a important. Cette performance est très importante dans le domaine de la vaccination, où on souhaite toujours protéger l'ensemble des sujets vaccinés, mais où les variabilités généralement observées entre les individus ne permettent pas d'assurer le même bénéfice à chacun des individus recevant le vaccin.

Ces résultats observés en présentant dans une même composition vaccinale un adjuvant comprenant à la fois un agoniste de récepteur Toll-like 4 et un agoniste des récepteurs Toll-like 7 et Toll-like 8, est d'autant plus surprenante que des tests réalisés en combinant un agoniste des récepteurs Toll-like 7 et Toll-like 8 et un agoniste d'un autre récepteur également présent sur les cellules présentatrices d'antigènes, n'ont pas permis d'améliorer les réponses par rapport aux réponses obtenues en utilisant comme adjuvant chacun des composés séparément.

Pour apprécier l'effet des compositions pharmaceutiques selon l'invention sur la réponse cellulaire, on effectue des comptages des cellules spléniques capables de produire de l'interféron γ par un test ELISPOT. Ce test est réalisé à la fois sur des cellules fraîches et sur des cellules restimulées.

Pour la réalisation du test, on cultive les cellules spléniques dans des plaques de culture cellulaire, à raison de 200 000 cellules par puits, en présence soit du milieu seul, soit de l'antigène TAT recombinante. Après 16 heures de culture, on révèle l'ELISPOT, i.e. que l'on compte le nombre de spots correspondant aux cellules sécrétant de l'interféron γ. Les résultats obtenus sont résumés dans les tableaux ci-après ; les valeurs indiquées sont les valeurs moyennes (par groupe de souris), des différences calculées pour chaque souris entre le nombre de spots comptés par million de cellules dans les puits ayant de la TAT recombinante et le nombre de spots comptés par million de cellules dans les puits n'ayant que du milieu.

Le tableau ci-après résume les résultats obtenus sur cellules fraîches.

| Composition immunostimulante testée | Nombre de spots par millions de cellules. |
|---|---|
| TAT à 20 µg | 7 |
| TAT à 20 µg + R-848 | 25 |
| TAT à 20 µg + ER804057 | 53 |
| TAT à 20µg + R-848 + ER804057 | 110 |

Le tableau ci-après résume les résultats obtenus sur cellules restimulées in vitro pendant 7 jours, en présence d'IL2, par un pool de peptides chevauchant couvrant entièrement la séquence de la protéine TAT.

| Composition immunostimulante testée | Nombre de spots par millions de cellules. |
|---|---|
| TAT à 20 µg | 33 |
| TAT à 20 µg + R-848 | 518 |
| TAT à 20 µg + ER804057 | 488 |
| TAT à 20µg + R-848 + ER804057 | 1005 |

En outre, on réalise en parallèle la mesure par un test ELISA de la sécrétion des cytokines IL5 et de l'interféron γ dans des surnageants de culture comprenant des splénocytes cultivés en présence ou non de TAT recombinante pendant 5 jours. Les résultats obtenus, exprimés en pg/ml sont résumés dans le tableau ci-après :

| Composition immunostimulante testée | IL-5 | INF-γ |
|---|---|---|
| TAT à 20 µg | 2893 | 7726 |
| TAT à 20 µg + R-848 | 152 | 8326 |
| TAT à 20 µg + ER804057 | 220 | 3886 |
| TAT à 20µg + R-848 + ER804057 | 167 | 13887 |

Ces résultats montrent l'effet particulièment intéressant obtenu sur la réponse TH1, grâce aux compositions selon la présente invention.

### 6. Préparation de suspensions de liposomes pour les tests de stimulation de cellules humaines.

On dispose de dipalmitoylphosphatidylcholine (DPPC) obtenue chez Avanti Polar Lipids (Alabaster, AL), et de 4-amino-2-ethoxymethyl-α, α, dimethyl-1-H-imidazo[4,5c]quinoleine-1-ethanol (R-848) fourni par la Société InVivogen. Ces composés se présentent sous forme de poudre.

9.92 mg de DPPC (13.5 µmoles) additionnés de 1 mg de R-848 (3.38 µmoles) sont dissous dans 2 ml d'un mélange chloroforme/méthanol 4 :1 (vol/vol). La solution est séchée dans un ballon en verre à l'aide d'un évaporateur rotatif de manière à laisser un film lipidique homogène sur les parois du ballon. Ce film est encore séché sous vide poussé pour éliminer toute trace de solvant résiduel puis repris dans 4 ml d'eau à 60°C. La suspension liposomale résultante est homogénéisée par agitation au vortex, sonication dans un bain à ultrasons puis extrudée séquentiellement, à l'aide d'un extrudeur Lipex thermostaté à 50°C, en un passage à travers une membrane de polycarbonate de porosité 0.8 µm, puis un passage à travers une membrane de porosité 0.4 µm et enfin un passage à travers une membrane de porosité 0.2 µm.

On obtient ainsi des liposomes de DPPC/R-848 (4:1 mol/mol) en eau à 2.48 mg/ml de DPPC et 250 µg/ml de R-848.

On dispose de dipalmitoylphosphatidylcholine (DPPC) obtenue chez Avanti Polar Lipids (Alabaster, AL) et de ER804057 fourni par la Société Eisai.

Ces composés se présentent sous forme de poudre.

19 mg de DPPC (25 µmoles) additionnés de 11 mg de ER804057 (6.7 µmoles) sont dissous dans 5ml d'un mélange chloroforme/méthanol 4 :1 (vol/vol). La solution est séchée dans un ballon en verre à l'aide d'un évaporateur rotatif de manière à laisser un film lipidique homogène sur les parois du ballon. Ce film est encore séché sous vide poussé pour éliminer toute trace de solvant résiduel puis repris dans 11 ml d'eau à 60°C. La suspension liposomale résultante est homogénéisée par agitation au vortex, sonication dans un bain à ultrasons puis extrudée séquentiellement, à l'aide d'un extrudeur Lipex thermostaté à 50°C, en un passage à travers une membrane de polycarbonate de porosité 0.8 µm, puis un passage à travers une membrane de porosité 0.4 µm et enfin un passage à travers une membrane de porosité 0.2 µm.

On obtient ainsi des liposomes de DPPC/ER804057 (4 :1 mol/mol) en eau à 1.72 mg/ml de DPPC et 1mg/ml de ER804057.

### 7. Test de stimulation de cellules humaines in vitro.

On évalue pour 4 donneurs indépendants la capacité des compositions selon l'invention à induire la maturation de cellules dendritiques dérivées de monocytes humains in vitro. Les monocytes sont obtenus à partir de cellules mononuclées du sang périphérique et sont cultivés pendant 5-6 jours en présence d'IL4 et de GM-CSF.

Ces cellules sont ensuite cultivées pendant 2 jours en présence de l'une des compositions suivantes :
- milieu de culture seul, servant de témoin négatif,
- liposomes R-848/DPPC préparés selon l'exemple 6 et dilués afin d'obtenir 2,96µg/ml de R-848,
- liposomes ER804057/DPPC préparés selon l'exemple 6 à raison de 0,1µg/ml.
- une combinaison des 2 préparations liposomales

On réalise ensuite une analyse phenotypique en cytométrie de flux, permettant de mesurer l'expression des marqueurs de maturation CD25, CD80 et CD83, ainsi qu'une mesure par ELISA des cytokines (TNF-α, IL6 et IL12p70) sécrétées par ces cellules.

Les résultats indiqués dans les tableaux ci-après représentent les moyennes calculées pour les 4 donneurs :

| | Pourcentage de cellules exprimant les marqueurs | | |
|---|---|---|---|
| | CD25 | CD80 | CD83 |
| milieu seul | 3 | 12 | 4 |
| R-848 | 25 | 34 | 19 |
| ER804057 | 35 | 46 | 15 |
| ER804057 + R-848 | 78 | 60 | 33 |

| | Quantité de cytokines en pg/ml | | |
|---|---|---|---|
| | TNF-α | IL 6 | IL12p70 |
| milieu seul | 61 | 77 | 10 |
| R-848 | 1727 | 8263 | 288 |
| ER804057 | 398 | 8349 | 22 |
| ER804057 + R-848 | 12041 | 69973 | 5304 |

Les résultats obtenus montrent la grande capacité des compositions selon l'invention à induire la sécrétion de cytokines indicatrices d'une réponse orientée TH1, telles que l'IL12p70 ; la synergie obtenue en combinant les 2 produits est remarquable. Les compositions selon l'invention sont donc particulièrement recommandées dans toutes les méthodes de traitement dans lesquelles on cherche à obtenir une réponse du système immunitaire orientée Th1, et notamment tous les cas où il est souhaitable d'induire la sécrétion de l'une des cytokines suivantes : TNF-α, IL-6 ou IL12p70.

## Revendications

1. Composition immunostimulante comprenant au moins un agoniste du récepteur Toll-like 7 ou du récepteur Toll-like 8, ainsi qu'un agoniste du récepteur Toll-like 4, **caractérisée en ce que** :
- l'agoniste du récepteur Toll-like 7 ou du récepteur Toll-like 8 est un dérivé d'imidazoquinoléine amine,
- l'agoniste du récepteur Toll-like 4 est le ER804057, et **en ce qu'**elle comprend en outre un antigène vaccinal autre qu'un ADN codant.

2. Composition immunostimulante selon la revendication précédente, **caractérisée en ce que** le dérivé d'imidazoquinoléine amine est le 4-amino-2-ethoxymethyl-α, α, dimethyl-1-H-imidazo[4,5c]quinoleine-1-ethanol.

3. Composition immunostimulante selon une des revendications précédentes, pour son utilisation comme médicament.

## Patentansprüche

1. Immunstimulierende Zusammensetzung, umfassend mindestens einen Agonisten des Toll-like 7 Rezeptors oder des Toll-like 8 Rezeptors sowie einen Agonisten des Toll-like 4 Rezeptors, **dadurch gekennzeichnet, dass**:
- es sich bei dem Agonisten des Toll-like 7 Rezeptors oder des Toll-like 8 Rezeptors um ein Imidazochinoleinaminderivat handelt,
- es sich bei dem Agonisten des Toll-like 4 Rezeptors um ER804057 handelt, und
dadurch, dass sie weiterhin ein Impfstoffantigen, bei dem es sich nicht um eine Codier-DNA handelt, umfasst.

2. Immunstimulierende Zusammensetzung nach dem vorhergehenden Anspruch, **dadurch gekennzeichnet, dass** es sich bei dem Imidazochinoleinaminderivat um 4-Amino-2-ethoxymethyl-α,α,dimethyl-1-H-imidazo[4,5c]chinolein-1-ethanol handelt.

3. Immunstimulierende Zusammensetzung nach einem der vorhergehenden Ansprüche zur Verwendung als Arzneimittel.

## Claims

1. Immunostimulant composition comprising at least one toll-like receptor 7 or toll-like receptor 8 agonist, and also a toll-like receptor 4 agonist, **characterized in that**:
- the toll-like receptor 7 or toll-like receptor 8 agonist is an imidazoquinoline amine derivative,
- the toll-like receptor 4 agonist is ER804057, and
**in that** it also comprises a vaccine antigen other than a coding DNA.

2. Immunostimulant composition according to the preceding claim, **characterized in that** the imidazoquinoline amine derivative is 4-amino-2-ethoxymethyl-α,α-dimethyl-1-H-imidazo[4,5-c]quinoline-1-ethanol.

3. Immunostimulant composition according to either of the preceding claims, for the use thereof as medicament.
